# EUROPEAN PATENT APPLICATION

(11) **EP 4 843 979 A1**
(43) Date of publication of application: **25.11.2026**
(21) Application number: 25178529.1
(22) Date of filing: 23.05.2025
(51) Int. Cl.: C07D 239/54, A01N 43/54

(54) **CRYSTALLINE FORMS OF CYCLOPROPYLMETHYL (2-{2-CHLORO-4-FLUORO-5-[3-METHYL-2,6-DIOXO-4-(TRIFLUOROMETHYL)-3,6-DIHYDROPYRIMIDIN-1(2H)-YL]PHENOXY}PHENOXY)ACETATE**

(71) Applicant: Bayer Aktiengesellschaft, 51373 Leverkusen (DE)
(72) Inventor: The designation of the inventor has not yet been filed
(74) Representative: BIP Patents

(57) **Abstract**

The present invention relates to the polymorphic form A of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate according to formula (1), to a process for its preparation, to agrochemical formulations comprising the novel crystalline form, and to its use in plant protection applications, especially to its use as a herbicide.

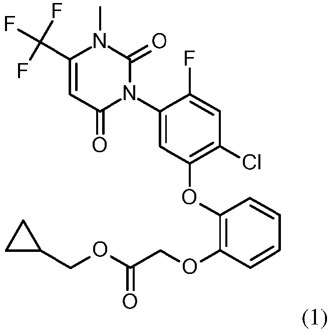

## Description

### Summary of the invention

The present invention relates to a novel crystalline form of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate according to formula (1), hereinafter referred to as polymorphic form A, to a process for its preparation, to agrochemical formulations comprising the novel crystalline form, to its use for the production of a formulation with increased stability and to its use in plant protection applications, especially to its use as a herbicide,

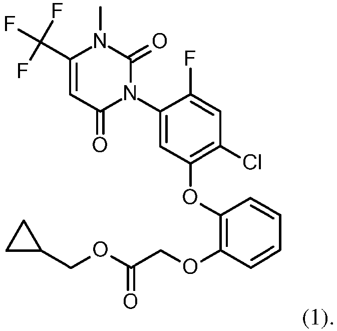

Cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate and a process for the manufacture is described in WO2024/078906.

Processes for the manufacture may result in amorphous forms, e.g. with sticky consistency, which may result in a difficult handling for the manufacture and/or use.

### Background

Polymorphism is the ability of a compound to crystallize in different crystalline phases with different arrangements and/or conformations of the molecules in the crystal lattice. Hence, polymorphs are different crystalline forms of the same pure chemical compound. On account of the different arrangement and/or conformation of molecules, polymorphs exhibit different physical, chemical and biological properties. Properties which may be affected include but are not limited to solubility, dissolution rate, stability, optical and mechanical properties, etc. The relative stability of a polymorph depends on its free energy, i.e. a more stable polymorph has a lower free energy. Under a defined set of experimental conditions only one polymorph has the lowest free energy. This polymorph is the thermodynamically stable form and all other polymorph(s) is (are) termed metastable form(s). A metastable form is one that is thermodynamically unstable but can nevertheless be prepared, isolated and analyzed as a result of its relatively slow rate of transformation.

The occurrence of active substances in different polymorphic forms (hereinafter also named as polymorphs or crystalline forms) is of decisive importance for the production in industrial scale as well as for the development of formulations containing the active substance, as unwanted phase change can lead to thickening and potentially solidification of the formulation and/or large crystals, which can lead to blockages in application equipment, e.g. in spray nozzles in agricultural application machinery. The knowledge of the existence of crystalline modifications and their properties is thus of high relevance. Each polymorph is characterized by a specific, uniform packing and arrangement of the molecules in the solid state. Nevertheless, it is generally not predictable whether a given chemical compound forms polymorphs at all and if so, which physical and biological properties the different polymorphs may have.

In addition pseudopolymorphic forms, named hydrates or solvates, can occur. A solvate is a crystalline molecular compound in which molecules of the solvent of crystallisation are incorporated into the host lattice, consisting of unsolvated molecules. A hydrate is a special case of a solvate, when the incorporated solvent is water. The presence of solvent molecules in the crystal lattice influences the intermolecular interactions and confers unique physical properties to each solvate. A solvate thus has its own characteristic values of internal energy, enthalpy, entropy, Gibbs free energy, and thermodynamic activity.

### Figures

Fig. 1a: X-ray powder diffractogram of polymorphic form A of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate
Fig. 1b: FT Raman spectrum of polymorphic form A of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate
Fig. 1c: IR spectrum of polymorphic form A of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate
Fig. 1d: DSC and TGA-thermograms of polymorphic form A of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate
Fig. 2a: X-ray powder diffractogram of mixture form A and B of cyclopropylmethyl (2-{2-chloro-4-fluoro-5-[3-methyl-2,6-dioxo-4-(trifluoromethyl)-3,6-dihydropyrimidin-1(2H)-yl]phenoxy }phenoxy)acetate
Fig. 2b: Comparison of X-ray powder diffractogram of mixture form A and B of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate (upper graph) to X-ray powder diffractogram of form A (lower graph)
Fig. 2c: DSC- and TGA-thermogram of mixture form A and B of cyclopropylmethyl (2-{2-chloro-4-fluoro-5-[3-methyl-2,6-dioxo-4-(trifluoromethyl)-3,6-dihydropyrimidin-1(2H)-yl]phenoxy }phenoxy)acetate

### Detailed description

In a first embodiment, the present invention relates to a novel crystalline form (hereinafter termed as form A) of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate according to formula (1),

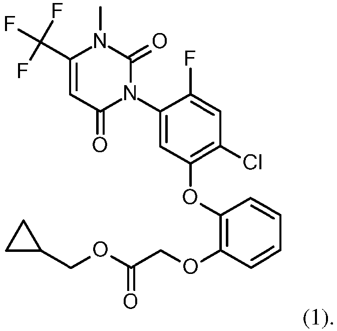

Object of the present invention is the provision of the thermodynamic stable polymorphic form of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate of formula (1) with beneficial physicochemical properties in solid state handling.

It has now surprisingly been found that by applying specific crystallisation conditions, a thermodynamic stable polymorphic form can be obtained, hereinafter referred to as "form A". In particular, form A displays good properties in bulk handling, purity and beneficial properties during the manufacturing of an agrochemical composition. In particular, form A displays a good stability in a temperature range between 0°C and 60°C. The increase in stability enhances the safety and quality of formulations comprising the compound of formula (1). Even further, the crystalline form A is well flowable and filterable.

The polymorphic form A of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate can be characterized by X-ray powder diffractometry on the basis of the respective diffraction diagrams, which are recorded at 25°C and with Cu-Kα 1 radiation (1.5406 Å). The polymorphic form A according to the present invention displays at least 3, often at least 5, in particular at least 7, more particularly at least 10, and especially all of the reflections quoted in the following table 1 as values:

**Table 1: X-ray reflections of polymorphic form A**

| Reflections [° 2Θ values] |
|---|
| x ± 0.2° |
| 20.7 |
| 20.4 |
| 19.5 |
| 23.3 |
| 27.4 |
| 21.5 |
| 25.3 |
| 22.4 |
| 11.1 |
| 7.0 |

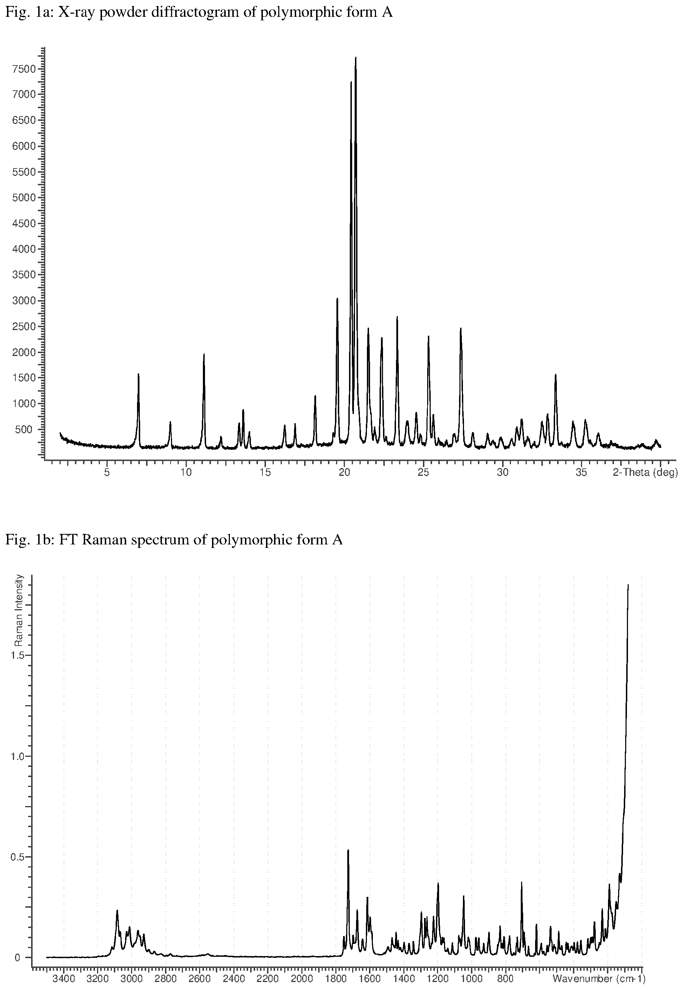

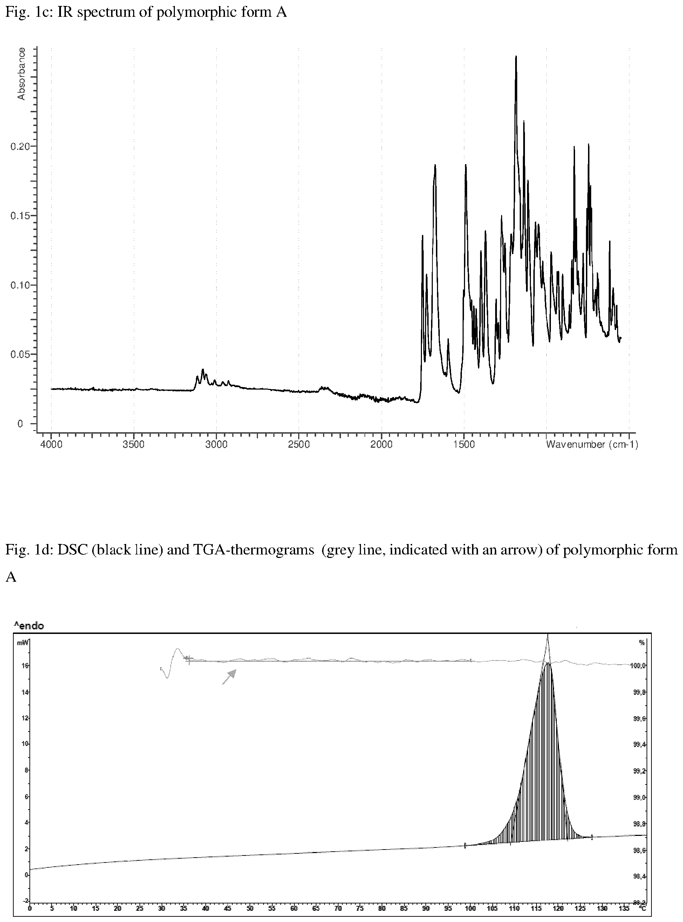

The polymorphic form A according to the present invention is further characterized by the X-ray powder diffractogram depicted in Fig. 1a.

In an embodiment, the polymorphic form A according to the present invention in a X-ray powder diffractogram at 25°C and Cu-Kα 1 radiation displays at least the following reflections, quoted as 2Θ value ± 0.2°: 20.7, 20.4 and 19.5.

Preferably, the polymorphic form A according to the present invention in a X-ray powder diffractogram at 25°C and Cu-Kα 1 radiation displays at least the following reflections, quoted as 2Θ value ± 0.2°: 20.7, 20.4, 19.5, 23.3 and 27.4.

Further preferably, the polymorphic form A according to the present invention in a X-ray powder diffractogram at 25°C and Cu-Kα 1 radiation displays at least the following reflections, quoted as 2Θ value ± 0.2°: 20.7, 20.4, 19.5, 23.3, 27.4, 21.5 and 25.3.

Even further preferably, the polymorphic form A according to the present invention in a X-ray powder diffractogram at 25°C and Cu-Kα 1 radiation displays at least the following reflections, quoted as 2Θ value ± 0.2°: 20.7, 20.4, 19.5, 23.3, 27.4, 21.5, 25.3, 22.4, 11.1 and 7.0.

Crystallographic studies on single crystals of form A revealed that the crystal structure is *monoclinic.* The unit cell has the space group P2_{1/n}.

**Table 2: Crystallographic characteristics of form A**

| Parameter | Form A |
|---|---|
| Crystal system | Monoclinic |
| Space group | P21/n |
| a | 13.6545(8) Å |
| b | 8.5730(5)Å |
| c | 20.0246(13) Å |
| α | 90° |
| β | 105.316(4)° |
| γ | 90° |
| Z | 4 |
| Density (calculated) | 1.595 g/cm³ |

| | |
|---|---|
| a, b, c = Length of the edges of the unit cell α , β, γ = Angles of the unit cell Z = Number of the molecules in the unit cell | |

The polymorphic form A of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate can be characterized by Raman spectroscopy on the basis of the respective spectrum, which is recorded at at room temperature and with a laser wavelength of 1064 nm and a resolution of 2 cm⁻¹, see Fig. 1b.

The polymorphic form A of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate can be characterized by infrared spectroscopy on the basis of the respective spectrum, which are recorded at room temperature using an universal diamond ATR device and a resolution of 2 cm⁻¹, see Fig. 1c.

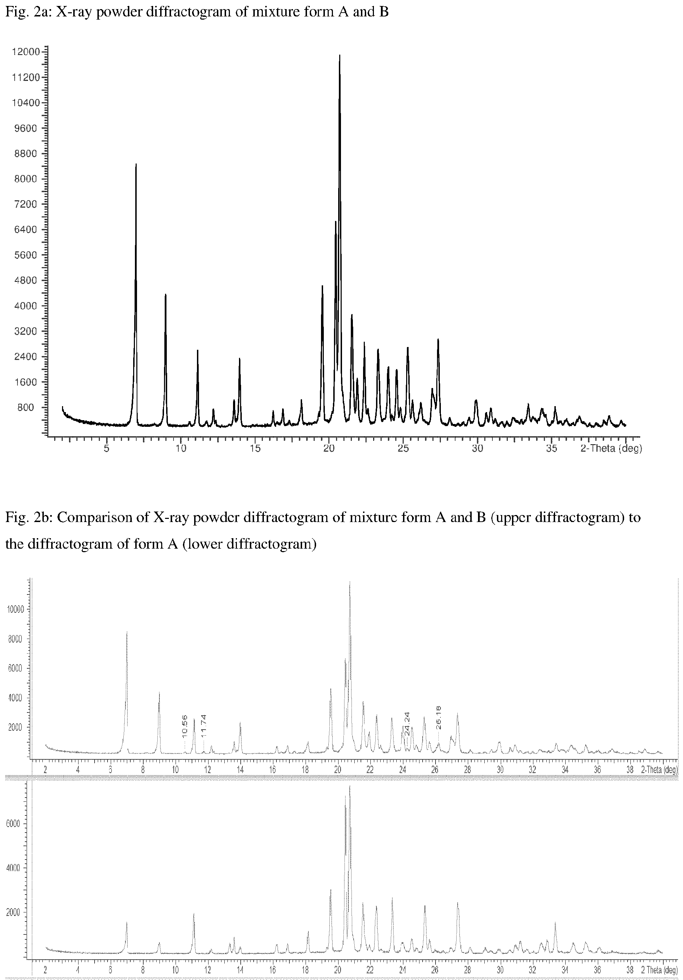

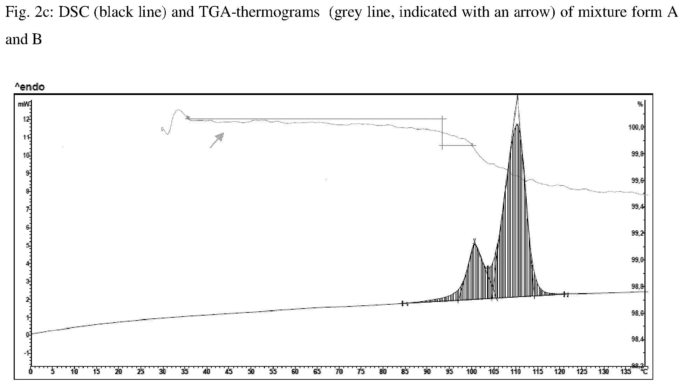

In addition to the polymorphic form A, one further polymorphic form, hereinafter denoted as form B (Fig. 2a-2d) has been identified. Form B only crystallises together with form A. Accordingly, this form is characterized in the following as mixture form A and B.

The mixture form A and B of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate can be characterized by X-ray powder diffractometry on the basis of the respective diffraction diagrams, which are recorded at 25°C and with Cu-Kα 1 radiation (1.5406 Å), see Fig. 2a and b.

Characteristic peaks of form B in the mixture are (° 2Θ values, X ± 0.2°): 10.56; 11.74; 24.24; 26.18.

In a further embodiment, the present invention is directed to a process for the production of the polymorphic form A, comprising the following steps:
a) diluting and/or suspending the compound of formula (1) in a suitable solvent or solvent mixture at the boiling temperature of the solvent or solvent mixture;
b) cooling the solution or slurry obtained in step a) to a temperature of less than 30 °C, preferably less than 25 °C, further preferably at room temperature,
c) incubation the solution or slurry at the temperature obtained in step b) until evaporation of the solvent or solvent mixture.

In step c), a precipitation agent can be added.

The chemical preparation of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate according to formula (1) in known from WO2024/078906. The compound of formula (1) as used in step a) can thus be prepared according to WO2024/078906, to which full reference is made hereby.

The compound of formula (1) in step a) can essentially be any known form of cyclopropylmethyl (2-{2-chloro-4-fluoro-5-[3-methyl-2,6-dioxo-4-(trifluoromethyl)-3,6-dihydropyrimidin-1(2H)-yl]phenoxy }phenoxy)acetate. This means that cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate can be used in amorphous form or in a mixture of different polymorphic forms or in a mixture containing an amorphous and one or more different polymorphic forms.

Suitable solvents or solvent mixtures which can be used to dilute and/or suspend the compound of formula (1) in step a) and from which the compound of formula (1) is obtained in polymorphic form A in step c), are polar or apolar organic solvents, or mixtures of water and polar solvents.

Preferred solvents are alkanes, or alkanols, or mixtures of alkanols and water, such as C₅-C₂₀ alkanes, or C₂-C₁₀ alkanoles.

Suitable solvents are, for example, diisopropyl ether, ethyl acetate, and methanol.

As precipitation agent in step c), n-heptane can be used.

The crystallization of polymorphic form A can be promoted or accelerated by seeding with seed crystals of form A.

The isolation of the polymorphic form A from the mother liquid is effected by common techniques known in the art, for example by filtration, centrifugation or by decanting.

Alternatively, the polymorphic form A is isolated from the solvent or solvent mixture by allowing the solution or slurry to stand at the crystallization conditions of step c) until at least 90 wt.-% of the solvent or solvent mixture is evaporated.

The isolated polymorphic form A can optionally be washed with any solvent, preferably with the solvent or solvent mixture used for crystallization, with water or with a mixture of the solvent or solvent mixture and water. The washing step can optionally be repeated, whereby washing with water often is the last washing step. The washing is typically performed at temperatures below 30°C, often below 25°C and in particular below 20°C, optionally at 0°C. In a further, optionally step, the crystals of polymorphic form A can be dried and then supplied for further processing.

By means of the crystallization according to the present invention, form A of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate is obtained with at least 85 %, in particular 90 %, and most preferably at least ≥ 95 %.

The content of form A according to the present invention may, for example, be analyzed by Raman spectroscopy. Based on calculated electronically mixed Raman spectra (mixed by a software calculator in 5 % steps) a calibration curve, using a PLS regression, may be generated. With this curve the proportional shares of the different forms may be calculated. Due to the calibration accuracy the content of 100 % of polymorphic form A is not excluded by the above wording.

Thus, a particular embodiment of the present invention relates to cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate, which consists of at least 85 % and often at least 90 % or at least ≥ 95 % of the polymorphic form A.

In a further embodiment, the present invention is directed to a plant protection agent in the form of customary formulations containing the polymorphic form A of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate.

The plant protection agent may additionally comprise one or more further active substance(s) selected from the group consisting of herbicides, insecticides, acaricides, fungicides, safeners and/or plant growth regulator as known in the art, e.g. as described in WO2024/078906, and to which disclosure full reference is made.

The plant protection agent may further comprise adjuvants which improve action, such as penetrants, e.g. vegetable oils, for example rapeseed oil, sunflower oil, mineral oils, for example paraffin oils, alkyl esters of vegetable fatty acids, for example rapeseed oil methyl ester or soya oil methyl ester, or alkanol alkoxylates and/or spreaders, for example alkylsiloxanes and/or salts, for example organic or inorganic ammonium or phosphonium salts, for example ammonium sulfate or diammonium hydrogenphosphate and/or retention promoters, for example dioctyl sulfosuccinate or hydroxypropylguar polymers and/or humectants, for example glycerol and/or fertilizers, for example ammonium-, potassium- or phosphorus-containing fertilizers.

Customary formulations are, for example, water-soluble liquids (SL), emulsion concentrates (EC), emulsions in water (EW), suspension concentrates (SC, SE, FS, OD), water-dispersible granules (WG), granules (GR) and capsule concentrates (CS); these and further possible formulation types are described, for example, by Crop Life International and in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576.

Preference is given to formulations or use forms comprising auxiliaries, for example extenders, solvents, spontaneity promoters, carriers, emulsifiers, dispersants, frost protection agents, biocides, thickeners and/or further auxiliaries, for example adjuvants. An adjuvant in this context is a component which enhances the biological effect of the formulation, without the component itself having any biological effect. Examples of adjuvants are agents which promote retention, spreading, attachment to the leaf surface or penetration.

These formulations are prepared in a known way, for example by mixing the compounds of the formula (I) with auxiliaries such as, for example, extenders, solvents and/or solid carriers and/or other auxiliaries such as, for example, surfactants. The formulations are produced either in suitable facilities or else before or during application.

The auxiliaries used may be substances suitable for imparting special properties, such as certain physical, technical and/or biological properties, to the formulation of the compounds of the formula (I), or to the use forms prepared from these formulations (for example ready-to-use pesticides such as spray liquors or seed dressing products).

In a preferred embodiment of the present invention, the plant protection agent contains more than 90 wt.%, and preferably more than 95 wt.%, of the polymorphic form A of the compound of the formula (1) based on the total amount of all forms of the compound of the formula (1) present in the composition.

It was now surprisingly found that the polymorphic form A has improved formulation properties, such as improved storage stability.

In a further embodiment, the present invention is therefore directed to the use of the polymorphic form A of the compound of formula (1) for the production of a herbicidal formulation.

In a further embodiment, the present invention is directed to the use of the polymorphic form A of the compound of formula (1) for combatting unwanted plant growth. In a particular embodiment, the present invention is directed to combatting unwanted plant growth in crops of useful plants, for example example cereal, such as wheat, barley, rye, oats, millet, rice, maniok and corn, or else in crops of sugarbeet, cotton, soya, rapeseed, potato, tomato, pea and other vegetable species. In a particular embodiment of the present invention, the useful plants are transgenic plants, such as plants tolerant to PPO inhibiting herbicides, as described, e.g. in WO2024/015949, US10370677, WO2017039969, US10563220, US10745712, WO2019118726.

In a further embodiment, the present invention is directed to a method for controlling harmful plants or for regulating the growth of plants, characterized in that an effective amount of the polymorphic form A or a plant protection agent as defined above containing the polymorphic form A, is applied to the plants, seeds of plants, the soil in which or on which the plants grow or the area under cultivation.

### Working examples

### Methods

All data which is part of the present application has been prepared according to the methods described below unless otherwise indicated. The samples used for measurement were directly used and did not undergo any further sample preparation.

### XRPD

X-Ray diffraction patterns were recorded at room temperature using XRD -diffractometers X'Pert PRO (PANalytical) and STOE STADI-P (radiation Cu K alpha 1, wavelength 1.5406 Å). All X-Ray reflections are quoted as °2Θ (theta) values (peak maxima) with a resolution of ± 0.2°.

### Raman

Raman spectra were recorded at room temperature using FT-Raman-spectrophotometers (model MultiRam) from Bruker. Resolution was 2 cm⁻¹. Measurements were performed in glass vials or aluminium discs.

### IR

IR-ATR-spectra were recorded at room temperature using a FT-IR-spectrophotometer (model Lumos or Tensor 37) with universal diamond ATR device from Bruker. Resolution was 2 cm⁻¹.

### TGA

TGA thermograms were recorded using thermobalances (model TGA/DSC 3+) from Mettler Toledo. The measurements were performed with a heating rate of 10 Kmin-1 using non-gastight aluminium pans. Flow gas was nitrogen. There was no sample preparation.

**Table 3: Physical characterization of polymorphic forms of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate Summary of XRPD Reflections, Raman and IR bands**

| XRPD Reflections (Peak maxima) [°2 Theta] |
|---|
| Polymorphic form A |
| 7.0 |
| 9.0 |
| 11.1 |
| 12.2 |
| 13.3 |
| 13.6 |
| 14.0 |
| 16.2 |
| 16.9 |
| 18.2 |
| 19.5 |
| 20.4 |
| 20.7 |
| 21.5 |
| 21.9 |
| 22.4 |
| 22.6 |
| 23.3 |
| 24.0 |
| 24.5 |
| 24.8 |
| 25.3 |
| 25.6 |
| 26.9 |
| 27.4 |
| 28.1 |
| 29.1 |
| 29.4 |
| 29.9 |
| 30.6 |
| 30.9 |
| 31.2 |
| 31.6 |
| 32.5 |
| 32.9 |
| 33.4 |
| 34.5 |
| 35.2 |
| 36.1 |

| Raman Bands [Peak maxima in cm⁻¹] |
|---|
| Polymorphic form A |
| 3116 |
| 3084 |
| 3067 |
| 3029 |
| 3012 |
| 2963 |
| 2952 |
| 2929 |
| 2901 |
| 2868 |
| 1751 |
| 1725 |
| 1697 |
| 1673 |
| 1642 |
| 1613 |
| 1596 |
| 1492 |
| 1469 |
| 1445 |
| 1433 |
| 1420 |
| 1399 |
| 1369 |
| 1344 |
| 1297 |
| 1276 |
| 1263 |
| 1224 |
| 1197 |
| 1171 |
| 1162 |
| 1141 |
| 1114 |
| 1075 |
| 1069 |
| 1047 |
| 1020 |
| 975 |
| 959 |
| 929 |
| 899 |
| 833 |
| 821 |
| 811 |
| 778 |
| 751 |
| 731 |
| 706 |
| 691 |
| 665 |
| 619 |
| 590 |
| 576 |
| 556 |
| 536 |
| 516 |
| 488 |
| 471 |
| 443 |
| 434 |
| 411 |
| 397 |
| 379 |
| 358 |
| 317 |
| 300 |
| 291 |
| 279 |
| 231 |
| 212 |
| 189 |
| 149 |
| 129 |

| IR Bands [Peak maxima in cm⁻¹] |
|---|
| Polymorphic form A |
| 3114 |
| 3082 |
| 3061 |
| 3009 |
| 2962 |
| 2926 |
| 1751 |
| 1728 |
| 1673 |
| 1596 |
| 1489 |
| 1454 |
| 1441 |
| 1427 |
| 1397 |
| 1370 |
| 1306 |
| 1293 |
| 1273 |
| 1251 |
| 1214 |
| 1185 |
| 1137 |
| 1112 |
| 1067 |
| 1050 |
| 1024 |
| 971 |
| 935 |
| 928 |
| 904 |
| 861 |
| 845 |
| 831 |
| 820 |
| 808 |
| 778 |
| 756 |
| 746 |
| 734 |
| 729 |
| 701 |
| 691 |
| 686 |
| 618 |
| 598 |
| 574 |

### Examples

### 1. Example 1

416 mg of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate was dissolved in 40 ml diisopropyl ether at its boiling temperature; the solution was filtered, cooled to room temperature and stored until evaporation of the solvent. N-Heptane was added as anti-solvent (100ml) during incubation.

The obtained crystals of polymorph A were isolated and analyzed by X-ray powder diffraction (XRPD), Raman and IR.

### 2. Example 2

404 mg of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate was dissolved in 40 ml ethyl acetate at its boiling temperature; the solution was filtered, cooled to room temperature and stored until evaporation of the solvent. N-Heptane was added as anti-solvent (100ml) during incubation.

The obtained crystals of polymorph A were isolated and analyzed by X-ray powder diffraction (XRPD), Raman and IR.

### 3. Example 3

408 mg of cyclopropylmethyl (2-{2-chloro-4-fluoro-5-[3-methyl-2,6-dioxo-4-(trifluoromethyl)-3,6-dihydropyrimidin-1(2H)-yl]phenoxy}phenoxy)acetate was dissolved in 40 ml methanol at its boiling temperature; the solution was filtered, cooled to room temperature and stored until evaporation of the solvent. Water was added as anti-solvent (100ml) during incubation.

The obtained crystals of polymorph A were isolated and analyzed by X-ray powder diffraction (XRPD), Raman and IR.

### 4. Example 4-6

Example 4-5 were conducted as examples 1-2, but the incubation/storing was conducted at -20°C instead of room temperature. A mixture of polymorph A and B was obtained.

## Claims

1. A crystalline form A of the compound of formula (1) 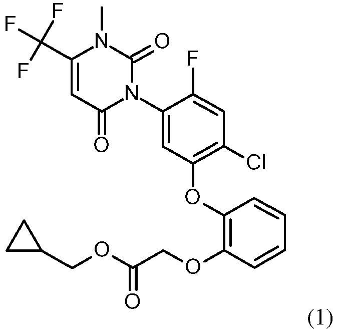 which in a X-ray powder diffractogram at 25°C and Cu-Kα 1 radiation displays at least the following reflections, quoted as 2Θ value ± 0.2°: 20.7, 20.4 and 19.5.

2. The form A of the compound of claim 1, which in a X-ray powder diffractogram at 25°C and with Cu-Kα 1 radiation displays at least the following reflections, quoted as 2Θ value ± 0.2°: 20.7, 20.4, 19.5, 23.3 and 27.4.

3. The form A of the compound of claim 1, which in a X-ray powder diffractogram at 25°C and with Cu-Kα 1 radiation displays at least the following reflections, quoted as 2Θ value ± 0.2°: 20.7, 20.4, 19.5, 23.3, 27.4, 21.5 and 25.3.

4. The form A of the compound of claim 1, which in a X-ray powder diffractogram at 25°C and with Cu-Kα 1 radiation displays at least the following reflections, quoted as 2Θ value ± 0.2°: 20.7, 20.4, 19.5, 23.3, 27.4, 21.5, 25.3, 22.4, 11.1 and 7.0.

5. A plant protection agent containing the crystalline form A of formula (1) according to any of claims 1 to 4, optionally further comprising one or more agriculturally acceptable additive customary for the formulation of plant protection agents, and/or one or more additional active substance(s) selected from the group consisting of herbicides, insecticides, acaricides, fungicides, safeners and/or plant growth regulator.

6. Use of the polymorphic form A according to any of claims 1 to 4 or of a plant protection agent according to claim 5 for the production of a formulation for controlling harmful plants.

7. Method for controlling harmful plants or for regulating the growth of plants, **characterized in that** an effective amount of the polymorphic form A according to any one of claims 1 to 4 or a plant protection agent according to claim 5 is applied to the plants, seeds of plants, the soil in which or on which the plants grow or the area under cultivation.
